(19) Europäisches Patentamt
European Patent Office
Office européen des brevets

(11) Veröffentlichungsnummer: **0 031 910**
B1

(12) # EUROPÄISCHE PATENTSCHRIFT

(45) Veröffentlichungstag der Patentschrift: **13.04.83**

(21) Anmeldenummer: **80107680.3**

(22) Anmeldetag: **06.12.80**

(51) Int. Cl.³: **C 07 C 87/62,** C 07 C 85/00, C 07 D 211/26, C 07 C 93/14, A 61 K 31/135, C 07 D 317/66, C 07 C 91/42, C 07 C 87/64, A 61 K 31/445

(54) **Anilinderivate, diese enthaltende pharmazeutische Zubereitungen und Verfahren zu ihrer Herstellung.**

(30) Priorität: **08.01.80 DE 3000441**

(43) Veröffentlichungstag der Anmeldung: **15.07.81 Patentblatt 81/28**

(45) Bekanntmachung des Hinweises auf die Patenterteilung: **13.04.83 Patentblatt 83/15**

(84) Benannte Vertragsstaaten: **AT BE CH DE FR GB IT LI NL SE**

(56) Entgegenhaltungen:
**DE-A-2 742 174**
**DE-A-2 929 181**

(73) Patentinhaber: **Merck Patent Gesellschaft mit beschränkter Haftung, Frankfurter Strasse 250, D-6100 Darmstadt (DE)**

(72) Erfinder: **Hausberg, Hans-Heinrich, Dr., Odenwaldstrasse 30, D-6105 Ober-Ramstadt (DE)**
Erfinder: **Uhl, Jürgen, Dr., Odenwaldstrasse 49, D-6104 Seeheim 1 (DE)**
Erfinder: **Minck, Klaus, Dr., Büchestrasse 8, D-6105 Ober-Ramstadt (DE)**
Erfinder: **Seyfried, Christoph, Dr., Am Landbach 9, D-6104 Seeheim-Jugenheim (DE)**

EP 0 031 910 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Anilinderivate, diese enthaltende pharmazeutische Zubereitungen und Verfahren zu ihrer Herstellung

Die Erfindung betrifft neue Anilinderivate der allgemeinen Formel I

$$Ar-N(CH_3)-R \qquad I$$

worin

Ar Phenyl oder eine durch F, Cl, Alkoxy mit bis zu 4 C-Atomen, Methylendioxy, OH, Trimethylen oder $CF_3$ substituierte Phenylgruppe,

R $-CH(C_6H_5)-CH_2CH_2-N(CH_3)-Z$ oder 2-(1-Methyl-2-piperidyl)-ethyl und H oder Methyl

bedeuten,
sowie ihre physiologisch unbedenklichen Säureadditionssalze.

Der Erfindung lag die Aufgabe zugrunde, neue Verbindungen aufzufinden, die zur Herstellung von Arzneimitteln verwendet werden können. Diese Aufgabe wurde durch die Bereitstellung der Verbindungen der Formel I gelöst.

Es wurde gefunden, daß die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem antidepressive Wirkungen. Im einzelnen kann eine reserpinantagonistische Wirkung (feststellbar z. B. an Mäusen gegenüber Reserpin in Anlehnung an die Methode von Askew, Life Science, Bd. 10 [1963], Seiten 725–730), eine antikataleptische Wirkung (feststellbar z. B. an Ratten gegenüber Tetrabenazin in Anlehnung an die Methode von Giurgea et al., Medicina Experimentalis, Bd. 9 [1963], Seiten 249–262) und eine antiptotische Wirkung (feststellbar z. B. gegenüber Reserpin in Anlehnung an die Methodik von Domenjoz und Theobald, Arch. int. pharmacodyn., Bd. 120 [1959], Seite 450 ff., mit der Bewertung nach Rubin et al., J. Pharmacol. Exp. Therap., Bd. 120 [1957], Seiten 125–136) nachgewiesen werden. Weiterhin kann die Wirkung des 5-Hydroxytryptophans bei Mäusen (Methode: ähnlich wie Ross et al., Acta pharmacol. et toxicol., Bd. 39 [1976], Seiten 152–166) potenziert werden und die zentralen Auswirkungen einer Erregung und Temperatursteigerung, die D-Amphetaminsulfat (z. B. 1,5 mg/kg s.c., 1 Stunde nach der Prüfsubstanz verabfolgt, die ebenfalls s.c. appliziert wird) und Aggregation (Zusammensetzung von 5 Ratten in einem Glas) auslösen (Methodik nach Müller-Calgan et al. in Zippel, H. P. (Ed.): Memory and Transfer of Information, Plenum Press, New York–London, 1973, Seiten 87–125), können erhöht und/oder verlängert werden. Die Substanzen haben einen Einfluß auf die biogenen Amine des ZNS. So führen sie z. B. in vitro zur Aufnahmehemmung von Noradrenalin, 5-Hydroxytryptamin und Dopamin (Methodik: Kannengießer et al., Biochem. Pharmacol., Bd. 22 [1973], Seiten 73–84) in Synaptosomen und hemmen in vivo (Methodik: Carlsson et al., Europ. J. Pharmacol., Bd. 5 [1969], Seiten 357–366; 367–373) die durch Tyraminderivate induzierte Katecholamin- und Serotonin-Freisetzung im Gehirn. Ferner antagonisieren die Verbindungen die durch p-Chloramphetamin hervorgerufene Serotoninspiegelsenkung bei Ratten bei oraler Applikation (Methodik: Fuller et al., Biochem. Pharmacol., Bd. 27 [1978], Seiten 193–198). Weiterhin treten spasmolytische Wirkungen auf, die nach hierfür geläufigen Methoden ermittelt werden können.

Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Gegenstand der Erfindung sind die Anilinderivate der Formel I sowie ihre physiologisch unbedenklichen Säureadditionssalze.

Im Rest Ar bedeutet Alkoxy vorzugsweise Methoxy, ferner auch Ethoxy, n-Propoxy, Isopropoxy, n-Butoxy, Isobutoxy, sek.-Butoxy oder tert.-Butoxy.

Im einzelnen bedeutet der Rest Ar vorzugsweise Fluorphenyl, Chlorphenyl, Methoxyphenyl, Hydroxyphenyl, 4- oder 5-Indanyl oder Trifluormethylphenyl, insbesondere p-Fluorphenyl, p-Chlorphenyl, o- oder p-Methoxyphenyl, 5-Indanyl oder p-Trifluormethylphenyl. Weiterhin kann Ar stehen für Phenyl, o- oder m-Fluorphenyl, o- oder m-Chlorphenyl, m-Methoxyphenyl, o-, m- oder p-Ethoxyphenyl, o-, m- oder p-n-Propoxyphenyl, o-, m- oder p-Isopropoxyphenyl, o-, m- oder p-n-Butoxyphenyl, o-, m- oder p-Isobutoxyphenyl, o-, m- oder p-sek.-Butoxyphenyl, o-, m- oder p-tert.-Butoxyphenyl, 2,3- oder 3,4-Methylendioxyphenyl, o-, m- oder p-Hydroxyphenyl, 4-Indanyl, o- oder m-Trifluormethylphenyl.

Der Rest R steht vorzugsweise für 1-Phenyl-3-dimethylamino-propyl.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat.

Die Verbindungen der Formel I können ein oder mehrere asymmetrische Kohlenstoffatome besitzen. Sie können daher als Racemate, falls mehrere asymmetrische Kohlenstoffatome vorhanden sind, auch als Gemische mehrerer Racemate sowie in verschiedenen optisch-aktiven Formen

vorliegen.

Gegenstand der Erfindung ist ferner ein Verfahren zur Herstellung der Verbindungen der Formel I sowie ihrer physiologisch unbedenklichen Säureadditionssalze, dadurch gekennzeichnet, daß man ein Amin der allgemeinen Formel II

$$Ar-NHCH_3 \qquad II$$

worin

Ar die angegebene Bedeutung hat,

oder eines seiner Salze mit einem Amin der allgemeinen Formel III

$$X-R \qquad III$$

worin

X Cl, Br, J oder OH bedeutet und
R die angegebene Bedeutung hat,

oder mit einem seiner reaktionsfähigen Derivate umsetzt
oder daß man ein Amin der allgemeinen Formel IV

$$Ar-Y \qquad IV$$

worin

Y $-NH-R$, N-Methyl-N-(1-phenyl-3-aminopropyl)-amino oder N-Methyl-N-[2-(2-piperidyl)-ethyl]-amino bedeutet und
Ar und R die angegebenen Bedeutungen haben,

methyliert
oder daß man ein Amin der allgemeinen Formel V

$$Ar-N(CH_3)-CH(C_6H_5)-CH_2-CH_2-X \qquad V$$

worin

Ar und X die angegebenen Bedeutungen haben,

oder eines seiner reaktionsfähigen Derivate mit einem Amin der allgemeinen Formel VI

$$H-N(CH_3)-Z \qquad VI$$

worin

Z die angegebene Bedeutung hat,

umsetzt
oder daß man eine Verbindung, die der allgemeinen Formel I entspricht, jedoch an Stelle von Wasserstoffatomen eine oder mehrere reduzierbare Gruppen und/oder eine oder mehrere C–C- und/oder C–N-Mehrfachbindungen enthält, mit einem reduzierenden Mittel behandelt
oder daß man eine Verbindung der allgemeinen Formel VII

$$Ar-N(CH_3)-CH(C_6H_5)-CH_2-CH_2-N(CH_3)-W \qquad VII$$

worin

W eine Aminoschutzgruppe bedeutet und
Ar die angegebene Bedeutung hat,

mit einem solvolysierenden Mittel behandelt
und/oder daß man gegebenenfalls in einer Verbindung der Formel I eine NH-Gruppe zur $N(CH_3)$-Gruppe methyliert und/oder eine Hydroxygruppe durch Behandeln mit einem alkylierenden Mittel in die entsprechende Alkoxygruppe umwandelt und/oder eine Alkoxygruppe unter Bildung

einer Hydroxygruppe spaltet und/oder eine erhaltene Base der Formel I durch Behandeln mit einer Säure in eines ihrer physiologisch unbedenklichen Säureadditionssalze umwandelt.

Die Herstellung der Verbindungen der Formel I erfolgt im übrigen nach an sich bekannten Methoden, wie sie in der Literatur (z. B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die Verbindungen der Formel I können durch Reaktion der Amine der Formel II oder ihrer Salze mit den Verbindungen der Formel III erhalten werden.

Die Amine der Formel II sind größtenteils bekannt und können nach an sich bekannten Methoden hergestellt werden, z. B. durch Methylierung der Aniline der Formel $Ar-NH_2$.

In den Verbindungen der Formeln III und V bedeutet der Rest X vorzugsweise Cl oder Br. Reaktionsfähige Derivate dieser Verbindungen sind insbesondere die reaktionsfähigen Ester der Alkohole der Formeln III und V (X = OH), vorzugsweise die entsprechenden Alkylsulfonate (worin die Alkylgruppe 1–6 C-Atome besitzt) und die entsprechenden Arylsulfonate (worin die Arylgruppe 6–10 C-Atome besitzt), z. B. die entsprechenden Methan-, Benzol-, p-Toluol- oder Naphthalin-1- oder -2-sulfonate.

Die Verbindungen der Formel III sind größtenteils bekannt und können nach an sich bekannten Methoden hergestellt werden. So ist 3-Dimethylamino-1-phenylpropan-1-ol z. B. erhältlich durch Mannich-Reaktion aus Acetophenon, Formaldehyd und Dimethylamin und anschließende Reduktion des erhaltenen Ketons, 1-Methyl-2-(2-hydroxyethyl)-piperidin beispielsweise durch N-Methylierung von 2-(2-Hydroxy-ethyl)-piperidin, die Verbindungen der Formel III (X = Cl, Br oder J) aus den Alkoholen mit anorganischen Halogeniden wie $SOCl_2$, $PBr_3$ oder HJ, die Sulfonate durch Veresterung der Alkohole mit den entsprechenden Sulfonylchloriden.

Man setzt die Verbindungen der Formeln II und III zweckmäßig um unter N-Alkylierungsbedingungen in Anwesenheit oder Abwesenheit eines inerten Lösungsmittels bei Temperaturen zwischen etwa 0 und etwa 250°, vorzugsweise zwischen etwa 60 und 180° und bei Drücken zwischen etwa 1 und 50 bar. Geeignete Lösungsmittel sind z. B. Kohlenwasserstoffe (wie Hexan, Benzol, Toluol oder Xylol), Ether (wie Diethylether, Diisopropylether, Tetrahydrofuran [THF], Dioxan oder Diethylenglykoldimethylether), Amide wie Dimethylformamid (DMF), Sulfoxide wie Dimethylsulfoxid, Alkohole (wie Methanol, Ethanol, Isopropanol oder n-Butanol). Falls erwünscht, kann ein Katalysator zugegen sein. Bei der Verwendung von Ausgangsstoffen der Formel III (X = Cl, Br oder J) empfiehlt sich der Zusatz einer Base wie NaOH, KOH, $Na_2CO_3$, $NaHCO_3$, Triethylamin oder Pyridin. Auch ein Überschuß des Anilinderivats der Formel II kann als Base und/oder Lösungsmittel dienen.

Die Anilinderivate der Formel I sind weiterhin durch Reaktion von Verbindungen der Formel IV mit Methylierungsmitteln erhältlich.

Die Ausgangsstoffe der Formel IV (Y = $-NH-R$) sind beispielsweise erhältlich durch Umsetzung von Anilinen der Formel $Ar-NH_2$ mit Verbindungen der Formel $X-R$ (III), diejenigen der Formel IV [Y = N-Methyl-N-(1-phenyl-3-amino-propyl)-amino] durch Anlagerung von Methylanilinderivaten der Formel $Ar-NH-CH_3$ an Zimtsäurenitril und anschließende Reduktion, diejenigen der Formel IV (Y = N-Methyl-N-[2-(2-piperidyl)-ethyl]-amino) durch Hydrogenolyse entsprechender 1-Benzyl-2-piperidylderivate.

Die Methylierung der Verbindungen der Formel IV erfolgt allgemein bei Temperaturen zwischen etwa 0 und etwa 150°, vorzugsweise mit Formaldehyd/Ameisensäure, zweckmäßig mit etwa 37%iger wässeriger Formaldehydlösung in Gegenwart überschüssiger Ameisensäure bei etwa 90–110°. Eine reduktive Alkylierung mit Formaldehyd/Natriumcyanborhydrid, zweckmäßig in Gegenwart eines inerten Lösungsmittels wie THF in schwach saurem Medium bei Temperaturen zwischen etwa 0 und 40°, ist ebenfalls gut möglich; als Zwischenprodukt entsteht wahrscheinlich der entsprechende Aldehyd-Ammoniak, der nicht isoliert zu werden braucht. Weiterhin eignen sich Umsetzungen mit Methylchlorid, -bromid, -jodid oder -p-toluolsulfonat, zweckmäßig unter den oben angegebenen Bedingungen für die N-Alkylierung.

Primäre Amine der Formel $Ar-N(CH_3)-CH(C_6H_5)-CH_2-CH_2-NH_2$ können mit Methanol in Gegenwart von Raney-Nickel bei erhöhter Temperatur zu den entsprechenden Monomethylaminen der Formel $Ar-N(CH_3)-CH(C_6H_5)-CH_2-CH_2-NH-CH_3$ methyliert werden.

Diejenigen Verbindungen der Formel I, in denen R die Gruppe $-CH(C_6H_5)-CH_2CH_2-N(CH_2)-Z$ bedeutet, sind weiterhin durch Reaktion von Aminen der Formel V oder ihrer reaktionsfähigen Derivate mit Aminen der Formel VI (Methylamin oder Dimethylamin) zugänglich.

Die Ausgangsstoffe der Formel V (X = OH) sind beispielsweise aus den Anilinderivaten der Formel $Ar-NH-CH_3$ (II) mit 3-Phenyl-3-chlor-propan-1-ol erhältlich, Verbindungen der Formel V (X = Cl, Br oder J) daraus durch Reaktion mit anorganischen Halogeniden wie $SOCl_2$, $PBr_3$ oder HJ, die entsprechenden Sulfonate durch Veresterung der Alkohole V (X = OH) mit Sulfonylchloriden.

Die Umsetzung der Verbindungen der Formeln V und VI erfolgt unter Alkylierungsbedingungen,

zweckmäßig in Gegenwart eines der genannten inerten Lösungsmittel bei Temperaturen zwischen etwa 20 und 140°, vorzugsweise 80 und 120°, vorteilhaft unter Druck (bis zu etwa 100 bar).

Die Anilinderivate der Formel I können ferner hergestellt werden durch Reduktion entsprechender Ausgangsstoffe, die zusätzlich reduzierbare Gruppen und/oder C—C- und/oder C—N-Doppel- oder -Dreifachbindungen enthalten.

Unter den reduzierbaren Ausgangsstoffen sind solche der allgemeinen Formel VIII bevorzugt.

$$Ar-N(CH_3)-Q \qquad VI$$

worin

Q $-CH(C_6H_5)-CH=CH-N(CH_3)-Z$, $-CH(C_6H_5)-CH_2-CO-N(CH_3)-Z$, $-CH(C_6H_5)-CH_2-CH_2-N(CHO)-Z$, 2-(1-Methyl-6-oxo-2-piperidyl)-ethyl oder die Gruppe

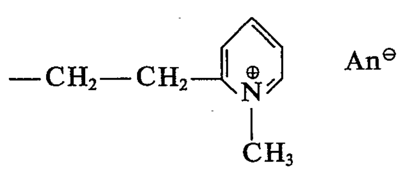

und

$An^{\ominus}$ ein Anion einer starken Säure bedeuten und

Ar die angegebene Bedeutung hat.

Die Verbindungen der Formel VIII können beispielsweise erhalten werden durch Umsetzung von Anilinen der Formel $Ar-NH-CH_3$ (II) mit Verbindungen der Formel $Q-X$ (worin X und Q die angegebenen Bedeutungen haben). Amide der Formel $Ar-N(CH_3)-CH(C_6H_5)-CH_2-CO-N(CH_3)-Z$ können auch durch Amidierung entsprechender Carbonsäureester der Formel $Ar-N(CH_3)-CH(C_6H_5)-CH_2-COOAlkyl$ (worin die Alkylgruppe 1—4 C-Atome besitzt) erhalten werden, N-Formylverbindungen der Formel $Ar-(CH_3)-CH(C_6H_5)-CH_2CH_2-N(CHO)-Z$ auch durch Formylierung der entsprechenden NH-Verbindungen der Formel

$$Ar-N(CH_3)-CH(C_6H_5)-CH_2CH_2-NH-Z.$$

Ferner sind z. B. Ausgangsstoffe geeignet, die der Formel I entsprechen, aber im Rest Ar an Stelle einer OH-Gruppe eine Benzyloxygruppe tragen.

Die Ausgangsstoffe der Formel VIII und die übrigen reduzierbaren Ausgangsstoffe können beispielsweise durch katalytische Hydrierung, mit nascierendem Wasserstoff, mit komplexen Metallhydriden oder mit Hilfe anderer chemischer Reduktionsmittel in die Verbindungen der Formel I umgewandelt werden. Die für die einzelnen Ausgangsstoffe geeigneten Reduktionsmethoden sind im allgemeinen von der Art der Gruppe abhängig und dem Fachmann nach den Angaben der Literatur geläufig. So können z. B. quartäre Pyridiniumsalze und olefinische Verbindungen besonders vorteilhaft katalytisch hydriert werden. Eine Reduktion der Säureamide erfolgt dagegen besonders vorteilhaft mit komplexen Metallhydriden oder mit Diboran.

Für katalytische Hydrierungen eignen sich beispielsweise Edelmetall-, Nickel- oder Kobaltkatalysatoren, ferner auch Mischkatalysatoren wie Kupferchromoxid. Als Edelmetalle kommen in erster Linie Platin und Palladium in Betracht, die auf Trägern (z. B. auf Kohle, Calciumcarbonat oder Strontiumcarbonat), als Oxide (z. B. Platinoxid) oder in feinteiliger Form vorliegen können. Nickel- und Kobaltkatalysatoren werden zweckmäßig als Raney-Metalle eingesetzt. Man kann zweckmäßig bei Drücken zwischen etwa 1 und 200 bar und bei Temperaturen zwischen etwa −80 und +150° hydrieren. Die Hydrierung erfolgt in Gegenwart eines inerten Lösungsmittels, z. B. eines Alkohols wie Methanol, Ethanol oder Isopropanol, einer Carbonsäure wie Essigsäure, eines Esters wie Ethylacetat, eines Ethers wie Tetrahydrofuran oder Dioxan. Man kann auch Lösungsmittelgemische verwenden, z. B. auch Wasser enthaltende Gemische. Bevorzugt ist eine Hydrierung unter milden Bedingungen, z. B. bei Temperaturen zwischen 0 und 30° unter Normaldruck.

Ferner können als Reduktionsmittel komplexe Metallhydride wie $LiAlH_4$, $NaBH_4$ oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Ether wie Diethylether, THF, Dioxan, 1,2-Dimethoxyethan oder Diglyme sowie Kohlenwasserstoffe wie Benzol. Für eine Reduktion mit $NaBH_4$ sind in erster Linie Alkohole wie Methanol oder Ethanol als Lösungsmittel geeignet. Nach dieser Methode reduziert man vorzugsweise bei Temperaturen zwischen etwa −80 und +150°, insbesondere zwischen etwa 20 und 120°.

Weiterhin ist als Reduktionsmethode die Umsetzung mit nascierendem Wasserstoff geeignet. Dieser kann beispielsweise durch Behandlung von Metallen mit Säuren oder Basen erzeugt werden.

So können z. B. die Systeme Zink/Säure, Zink/Alkalilauge, Eisen/Säure oder Zinn/Säure verwendet werden. Als Säuren eignen sich z. B. Salzsäure oder Essigsäure. Auch ein Alkalimetall wie Natrium in einem Alkohol wie Ethanol, Isopropanol, n-Butanol, Amylalkohol, Isoamylalkohol oder in Phenol kann als Reduktionsmittel verwendet werden, ferner z. B. eine Aluminium-Nickel-Legierung in alkalisch-wässeriger oder alkalisch-wässerig-alkoholischer Lösung sowie Natrium- oder Aluminiumamalgam in wässerig-alkoholischer oder wässeriger Lösung. Bei diesen Methoden liegen die Reaktionstemperaturen zwischen etwa 0 und etwa 150°, vorzugsweise zwischen etwa 20 und 120°.

Anilinderivate der Formel $Ar-N(CH_3)-CH(C_6H_5)-CH_2CH_2-NH-CH_3$ sind besonders vorteilhaft erhältlich durch solvolytische, vorzugsweise hydrolytische Abspaltung einer Aminoschutzgruppe aus entsprechenden geschützten Verbindungen der Formel VIII.

Der Ausdruck »Aminoschutzgruppe« ist allgemein bekannt und bezieht sich auf Gruppen, die geeignet sind, eine sekundäre Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Da die Aminoschutzgruppe bei der Solvolyse entfernt wird, ist ihre Art und Größe nicht kritisch. Bevorzugte Aminoschutzgruppen sind Acylgruppen mit insbesondere 1–20, vorzugsweise 1–8 C-Atomen. Der Ausdruck »Acylgruppe« ist in diesem Zusammenhang in weitestem Sinne aufzufassen. Er umschließt Acylgruppen, die von aliphatischen, araliphatischen, aromatischen oder heterocyclischen Carbonsäuren oder Sulfonsäuren abgeleitet sind, ferner z. B. Alkoxycarbonyl-, Aryloxycarbonyl- und Arylkoxycarbonylgruppen. Im einzelnen bevorzugte Aminoschutzgruppen sind Formyl, Acetyl, Trifluoracetyl und Benzoyl.

Die Ausgangsstoffe der Formel VII sind z. B. erhältlich durch Reaktion von Methylanilinen der Formel $Ar-NH-CH_3$ mit Verbindungen der Formel $X-CH(C_6H_5)-CH_2-CH_2-N(CH_3)-W$ oder deren reaktionsfähigen Derivaten, worin Ar, X und W die angegebenen Bedeutungen haben.

Die solvolytische Spaltung der Verbindungen der Formel VII gelingt zweckmäßig bei Temperaturen zwischen etwa 0 und etwa 120° durch Einwirkung eines Lösungsmittels wie Wasser (Hydrolyse) oder eines Alkohols mit vorzugsweise 1–4 C-Atomen (Alkoholyse) in Gegenwart eines sauren oder basischen Katalysators, z. B. einer Mineralsäure wie Schwefelsäure oder Salzsäure, eines Metallhydroxids wie Natrium-, Kalium-, Calcium-, Barium-, Blei- oder Silberhydroxid oder eines Metall- oder Ammoniumsalzes wie Natrium- oder Kaliumcarbonat oder Ammoniumchlorid. Als Alkohole eignen sich vorzugsweise Methanol, Ethanol oder Isopropanol; man kann auch Gemische von Wasser mit einem dieser Alkohole verwenden. Für die bevorzugten Formylderivate der Formel VII ($W=CHO$) verwendet man zweckmäßig wässerig-ethanolische KOH-Lösung in der Siedehitze als solvolysierendes Mittel.

Sekundäre Amine der Formel $Ar-N(CH_3)-CH(C_6H_5)-CH_2-CH_2-NH-CH_3$ können, falls erwünscht, nach einer der oben angegebenen Methoden zu den entsprechenden tertiären Aminen der Formel $Ar-N(CH_3)-CH(C_6H_5)-CH_2-CH_2-N(CH_3)$ methyliert werden.

Falls erwünscht, kann ein Phenol der Formel I (Ar = eine durch eine OH-Gruppe substituierte Phenylgruppe) alkyliert werden, wobei man eine Verbindung der Formel I (Ar = eine durch eine Alkoxygruppe substituierte Phenylgruppe) erhält. Als Alkylierungsmittel eignen sich z. B. Alkylhalogenide, wie Methylchlorid, -bromid oder -jodid, Dialkylsulfate wie Dimethylsulfat und Sulfonsäurealkylester wie Methyl-p-toluolsulfonat. Die O-Alkylierung wird zweckmäßig bei Temperaturen zwischen etwa 0 und etwa 150°, vorzugsweise zwischen 20 und 100°, in einem inerten Lösungsmittel vorgenommen, z. B. einem Alkohol wie Methanol oder Ethanol, einem Kohlenwasserstoff wie Benzol, einem Amid wie DMF, einem Ether wie THF oder einem Amin wie Pyridin. Verwendet man Halogenide, so arbeitet man zweckmäßig in Gegenwart einer Base wie NaOH, KOH, Triethylamin oder Pyridin, wobei ein Überschuß dieser Base als Lösungsmittel dienen kann.

Ether der Formel I (Ar = eine durch eine Alkoxygruppe substituierte Phenylgruppe) können nach Methoden, die aus der Literatur bekannt sind, gespalten werden. Dabei entstehen die entsprechenden Phenole der Formel I (Ar = eine durch eine OH-Gruppe substituierte Phenylgruppe). Zum Beispiel kann man die Ether spalten durch Behandeln mit HBr oder HJ in wässeriger oder essigsaurer Lösung, durch Erhitzen mit Lewis-Säuren wie $AlCl_3$ oder Bortrihalogeniden oder durch Verschmelzen mit Pyridin- oder Anilinhydrohalogeniden, vorzugsweise Pyridinhydrochlorid, bei etwa 150–250°.

Eine erhaltene Base der Formel I kann mit einer Säure in das zugehörige Säureadditionssalz übergeführt werden. Für die Umsetzung eignen sich Säuren, die physiologisch unbedenkliche Salze liefern. So können anorganische Säuren verwendet werden, z. B. Schwefelsäure, Halogenwasserstoffsäuren, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Apfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend eine Verbindung der Formel I und/oder eines ihrer physiologisch unbedenklichen Säureadditionssalze. Diese Zubereitungen können als Arzneimittel in der Human- oder Veterinärmedizin eingesetzt werden. Als Trägersubstanzen kommen organische oder anorganische Stoffe in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromatstoffe enthalten. Sie können, falls erwünscht, auch einen oder mehrere weitere Wirkstoffe enthalten, z. B. ein oder mehrere Vitamine.

Gegenstand der Erfindung ist ferner die Verwendung der Verbindungen der Formel I und ihrer physiologisch unbedenklichen Säureadditionssalze bei der Bekämpfung von Krankheiten, insbesondere von Depressionen verschiedener Ätiologie und Symptomatologie, sowie ihre Verwendung bei der therapeutischen Behandlung des menschlichen oder tierischen Körpers.

Dabei werden die erfindungsgemäßen Substanzen in der Regel in Analogie zu bekannten, im Handel befindlichen Psychopharmaka (z. B. Imipramin) verabreicht, vorzugsweise in Dosierungen zwischen etwa 2 und 500 mg, insbesondere zwischen 10 und 50 mg pro Dosierungseinheit. Die tägliche Dosierung liegt vorzugsweise zwischen etwa 0,05 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die Temperaturen sind vor- und nachstehend in °C angegeben.

In den nachfolgenden Beispielen bedeutet »übliche Aufarbeitung«:

Man gibt Natronlauge hinzu, extrahiert mit einem organischen Lösungsmittel wie Benzol, Chloroform oder Dichlormethan, trennt ab, trocknet die organische Phase über Natriumsulfat, filtriert, dampft ein und reinigt durch Chromatographie und/oder Kristallisation.

Die Rf-Werte wurden an Kieselgel mit Toluol/Triethylamin 8 : 2 als Lösungsmittel erhalten.

## Beispiel 1

Ein Gemisch von 14,2 g p-Chlor-N-methyl-anilin, 23,4 g 1-Phenyl-3-dimethylamino-propylchlorid-hydrochlorid (oder 32,3 g 1-Phenyl-3-dimethylamino-propylbromid-hydrobromid), 27,6 g $K_2CO_3$ und 250 ml DMF wird 12 Stunden gekocht. Nach dem Abkühlen und üblicher Aufarbeitung erhält man p-Chlor-N-methyl-N-(1-phenyl-3-dimethylaminopropyl)-anilin. Dihydrochlorid, F. 164°.

## Beispiele 2 bis 20

Analog Beispiel 1 erhält man aus den entsprechenden N-Methyl-anilinen:

2. N-Methyl-N-(1-phenyl-3-dimethylaminopropyl)-anilin.
3. o-Fluor-N-methyl-N-(1-phenyl-3-dimethylaminopropyl)-anilin.
4. m-Fluor-N-methyl-N-(1-phenyl-3-dimethylaminopropyl)-anilin.
5. p-Fluor-N-methyl-N-(1-phenyl-3-dimethylaminopropyl)-anilin, Dihydrochlorid, F. 204°.
6. o-Chlor-N-methyl-N-(1-phenyl-3-dimethylaminopropyl)-anilin.
7. m-Chlor-N-methyl-N-(1-phenyl-3-dimethylaminopropyl)-anilin.
8. o-Methoxy-N-methyl-N-(1-phenyl-3-dimethylaminopropyl)-anilin, Oxalat, F. 139°; Fumarat, F. 114°.
9. m-Methoxy-N-methyl-N-(1-phenyl-3-dimethylaminopropyl)-anilin.

10. p-Methoxy-N-methyl-N-(1-phenyl-3-dimethylaminopropyl)-anilin, Dihydrochlorid, F. 194°.
11. p-n-Butoxy-N-methyl-N-(1-phenyl-3-dimethylaminopropyl)-anilin.
12. 3,4-Methylendioxy-N-methyl-N-(1-phenyl-3-dimethylaminopropyl)-anilin, Dihydrochlorid, F. 218–220°.
13. o-Hydroxy-N-methyl-N-(1-phenyl-3-dimethylaminopropyl)-anilin.
14. m-Hydroxy-N-methyl-N-(1-phenyl-3-dimethylaminopropyl)-anilin.
15. p-Hydroxy-N-methyl-N-(1-phenyl-3-dimethylaminopropyl)-anilin.
16. o-Trifluormethyl-N-methyl-N-(1-phenyl-3-dimethylaminopropyl)-anilin.
17. m-Trifluormethyl-N-methyl-N-(1-phenyl-3-dimethylaminopropyl)-anilin.
18. p-Trifluormethyl-N-methyl-N-(1-phenyl-3-dimethylaminopropyl)-anilin.
19. 4-(N-Methyl-N-1-phenyl-3-dimethylamino-propylamino)-indan.
20. 5-(N-Methyl-N-1-phenyl-3-dimethylamino-propylamino)-indan, Dihydrochlorid, F. 184°; Oxalat, F. 185–186°.

Beispiele 21 bis 40

Analog Beispiel 1 bis 20 erhält man mit 1-Methyl-2-(2-chlorethyl)-piperidin:

21. N-Methyl-N-[2-(1-methyl-2-piperidyl)-ethyl]-anilin.
22. o-Fluor-N-methyl-N-[2-(1-methyl-2-piperidyl)-ethyl]-anilin.
23. m-Fluor-N-methyl-N-[2-(1-methyl-2-piperidyl)-ethyl]-anilin.
24. p-Fluor-N-methyl-N-[2-(1-methyl-2-piperidyl)-ethyl]-anilin.
25. o-Chlor-N-methyl-N-[2-(1-methyl-2-piperidyl)-ethyl]-anilin.
26. m-Chlor-N-methyl-N-[2-(1-methyl-2-piperidyl)-ethyl]-anilin.
27. p-Chlor-N-methyl-N-[2-(1-methyl-2-piperidyl)-ethyl]-anilin, Rf 0,38.
28. o-Methoxy-N-methyl-N-[2-(1-methyl-2-piperidyl)-ethyl]-anilin.
29. m-Methoxy-N-methyl-N-[2-(1-methyl-2-piperidyl)-ethyl]-anilin.
30. p-Methoxy-N-methyl-N-[2-(1-methyl-2-piperidyl)-ethyl]-anilin, Hydrochlorid, F. 138°.
31. p-n-Butoxy-N-methyl-N-[2-(1-methyl-2-piperidyl)-ethyl]-anilin.
32. 3,4-Methylendioxy-N-methyl-N-[2-(1-methyl-2-piperidyl)-ethyl]-anilin.
33. o-Hydroxy-N-methyl-N-[2-(1-methyl-2-piperidyl)-ethyl]-anilin.
34. m-Hydroxy-N-methyl-N-[2-(1-methyl-2-piperidyl)-ethyl]-anilin.
35. p-Hydroxy-N-methyl-N-[2-(1-methyl-2-piperidyl)-ethyl]-anilin.
36. o-Trifluormethyl-N-methyl-N-[2-(1-methyl-2-piperidyl)-ethyl]-anilin.
37. m-Trifluormethyl-N-methyl-N-[2-(1-methyl-2-piperidyl)-ethyl]-anilin.
38. p-Trifluormethyl-N-methyl-N-[2-(1-methyl-2-piperidyl)-ethyl]-anilin, Rf 0,42.
39. 4-[N-Methyl-N-(2-(1-methyl-2-piperidyl)-ethyl)-amino]-indan.
40. 5-[N-Methyl-N-(2-(1-methyl-2-piperidyl)-ethyl)-amino]-indan.

Beispiele 41 bis 60

Analog Beispiel 1 bis 20 erhält man mit 1-Phenyl-3-methyl-aminopropylchlorid:

41. N-Methyl-N-(1-phenyl-3-methylaminopropyl)-anilin.
42. o-Fluor-N-methyl-N-(1-phenyl-3-methylaminopropyl)-anilin.
43. m-Fluor-N-methyl-N-(1-phenyl-3-methylaminopropyl)-anilin.
44. p-Fluor-N-methyl-N-(1-phenyl-3-methylaminopropyl)-anilin.
45. o-Chlor-N-methyl-N-(1-phenyl-3-methylaminopropyl)-anilin.
46. m-Chlor-N-methyl-N-(1-phenyl-3-methylaminopropyl)-anilin.
47. p-Chlor-N-methyl-N-(1-phenyl-3-methylaminopropyl)-anilin.
48. o-Methoxy-N-methyl-N-(1-phenyl-3-methylaminopropyl)-anilin.
49. m-Methoxy-N-methyl-N-(1-phenyl-3-methylaminopropyl)-anilin.
50. p-Methoxy-N-methyl-N-(1-phenyl-3-methylaminopropyl)-anilin.
51. p-N-Butoxy-N-methyl-N-(1-phenyl-3-methylaminopropyl)-anilin.
52. 3,4-Methylendioxy-N-methyl-N-(1-phenyl-3-methylaminopropyl)-anilin.
53. o-Hydroxy-N-methyl-N-(1-phenyl-3-methylaminopropyl)-anilin.
54. m-Hydroxy-N-methyl-N-(1-phenyl-3-methylaminopropyl)-anilin.
55. p-Hydroxy-N-methyl-N-(1-phenyl-3-methylaminopropyl)-anilin.
56. o-Trifluormethyl-N-methyl-N-(1-phenyl-3-methylaminopropyl)-anilin.
57. m-Trifluormethyl-N-methyl-N-(1-phenyl-3-methylaminopropyl)-anilin.
58. p-Trifluormethyl-N-methyl-N-(1-phenyl-3-methylaminopropyl)-anilin.

59. 4-(N-Methyl-N-1-phenyl-3-methylamino-propyl-amino)-indan.
60. 5-(N-Methyl-N-1-phenyl-3-methylamino-propyl-amino)-indan.

### Beispiel 61

Zu 29 g p-Chlor-N-(1-phenyl-3-dimethylamino-propyl)-anilin (erhältlich aus p-Chloranilin und 1-Phenyl-3-dimethylamino-propylchlorid) gibt man unter Eiskühlung 23 g 85%ige Ameisensäure und danach 9,9 g 37%ige wässerige Formaldehydlösung. Das Gemisch wird 5 Stunden auf 100° erhitzt, angesäuert und eingeengt, der Rückstand wie üblich aufgearbeitet. Man erhält p-Chlor-N-methyl-N-(1-phenyl-3-dimethylamino-propyl)-anilin. Dihydrochlorid, F. 164°.

### Beispiele 62 bis 100

Analog Beispiel 61 erhält man durch Methylierung der entsprechenden sekundären Anilinderivate die in den Beispielen 2 bis 40 angegebenen tertiären Anilinderivate.

### Beispiel 101

Man löst 25,1 g p-Chlor-N-[2-(1-methyl-2-piperidyl)-ethyl]-anilin in 200 ml THF und 100 ml Wasser, gibt Salzsäure bis pH 5 hinzu und versetzt mit 120 ml 37%iger wässeriger Formaldehydlösung. Nach 15 Minuten Rühren wird eine Lösung von 18,9 g $Na(CN)BH_3$ in 120 ml THF zugetropft. Dabei wird durch Zugabe von HCl der pH auf 6 gehalten. Man rührt noch 2 Stunden bei 20°, säuert mit konzentrierter Salzsäure an, wäscht mit Ether, macht alkalisch, arbeitet wie üblich auf und erhält p-Chlor-N-methyl-N-[2-(1-methyl-2-piperidyl)-ethyl]-anilin, Rf 0,38.

### Beispiele 102 bis 140

Analog Beispiel 101 erhält man durch Umsetzung der entsprechenden sekundären Anilinderivate mit Formaldehyd/$Na(CN)BH_3$ die in den Beispielen 1 bis 26 sowie 28 bis 40 angegebenen tertiären Anilinderivate.

### Beispiel 141

Ein Gemisch aus 31,1 g 1-Amino-3-phenyl-3-(p-chlor-N-methylanilino)-propan-hydrochlorid (erhältlich durch Anlagerung von p-Chlor-N-methylanilin an Zimtaldehyd, Reaktion des erhaltenen 3-Phenyl-3-(p-chlor-N-methylanilino)-propanals mit $NH_3$ zum Imin und Hydrierung), 50 ml Ameisensäure, 7 g Natriumformiat und 40 ml 40%iger Formaldehyd-Lösung wird 3 Stunden auf 60° und danach 12 Stunden auf 100° erhitzt. Nach üblicher Aufarbeitung erhält man p-Chlor-N-methyl-N-(1-phenyl-3-dimethylaminopropyl)-anilin. Dihydrochlorid, F. 164°.

### Beispiele 142 bis 160

Analog Beispiel 141 erhält man aus den entsprechenden 1-Amino-3-phenyl-3-N-methylanilino-propanen mit Ameisensäure/Natriumformiat/Formaldehyd die in den Beispielen 2 bis 20 beschriebenen Verbindungen.

### Beispiel 161

Man löst 27,5 g 1-Amino-3-phenyl-3-(p-chlor-N-methylanilino)-propan in 500 ml Methanol, versetzt mit 5 g Raney-Nickel und schüttelt das Gemisch 15 Stunden bei 160° im Bombenrohr. Nach Abkühlen, Filtration und Eindampfen arbeitet man wie üblich auf und erhält p-Chlor-N-methyl-N-(1-phenyl-3-methylaminopropyl)-anilin.

### Beispiele 162 bis 180

Analog Beispiel 161 erhält man durch Methylierung aus den entsprechenden primären Aminen die in den Beispielen 41 bis 46 sowie 48 bis 60 angegebenen sekundären Amine.

### Beispiel 181

Analog Beispiel 141 erhält man aus p-Chlor-N-methyl-N-[2-(2-piperidyl)-ethyl]-anilin mit Ameisensäure/Natriumformiat/Formaldehyd das p-Chlor-N-methyl-N-[2-(1-methyl-2-piperidyl)-ethyl]-anilin, Rf 0,38.

### Beispiele 182 bis 200

Analog Beispiel 181 erhält man aus den entsprechenden N-Methyl-N-[2-(2-piperidyl)-ethyl]-anilinen mit Ameisensäure/Natriumformiat/Formaldehyd die in den Beispielen 20 bis 26 und 28 bis 40 beschriebenen Verbindungen.

### Beispiel 201

Eine Lösung von 4,30 g 1-p-Toluolsulfonyloxy-3-phenyl-3-(p-chlor-N-methyl-anilino)-propan (erhältlich durch Reaktion von p-Chlor-N-methyl-anilin mit 3-Chlor-3-phenyl-propanol und Tosylierung) und 30 g Dimethylamin in 100 ml Methanol wird im Autoklaven 2 Stunden auf 120° erhitzt. Nach dem Abkühlen und üblicher Aufarbeitung erhält man p-Chlor-N-methyl-N-(1-phenyl-3-dimethylamino-propyl)-anilin. Dihydrochlorid, F. 164°.

### Beispiele 202 bis 240

Analog Beispiel 201 erhält man durch Umsetzung der entsprechenden 1-p-Toluolsulfonyloxy-, 1-Chlor- oder 1-Brom-3-phenyl-3-N-methyl-anilino-propane mit Dimethylamin die in den Beispielen 2 bis 20 angegebenen Verbindungen und mit Methylamin die in den Beispielen 41 bis 60 angegebenen Verbindungen.

### Beispiel 241

Ein Gemisch aus 27,9 g 3-Phenyl-3-(N-methyl-N-5-indanylamino)-propanal (erhältlich aus 5-Methylaminoindan und Zimtaldehyd), 100 g Dimethylamin und 300 ml Ethanol wird 12 Stunden in Gegenwart von 2 g 5%iger Pd-Kohle bei 100° und 5 bar hydriert. Als Zwischenprodukt entsteht vermutlich 1-Dimethylamino-3-phenyl-3-N-methyl-N-(5-indanyl)-amino-propen, das nicht isoliert wird. Nach Eindampfen und üblicher Aufarbeitung erhält man 5-(N-Methyl-N-1-phenyl-3-dimethylamino-propylamino)-indan. Dihydrochlorid, F. 184°.

### Beispiele 242 bis 260

Analog Beispiel 241 erhält man durch Umsetzung der entsprechenden 3-Phenyl-3-N-methylanilinopropanale mit Dimethylamin und Wasserstoff die in den Beispielen 1 bis 19 angegebenen Verbindungen.

### Beispiel 261

Zu einer Suspension von 7,6 g $LiAlH_4$ in 250 ml absolutem THF tropft man unter Rühren eine Lösung von 30 g 3-Phenyl-3-(p-fluor-N-methyl-anilino)-propansäure-N,N-dimethylamid (erhältlich durch Anlagerung von p-Fluor-N-methylanilin an Zimtsäure-ethylester und Umsetzen des erhaltenen 3-Phenyl-3-(p-fluor-N-methyl-anilino)-propansäureethylesters mit Dimethylamin) in 500 ml THF, kocht 16 Stunden, versetzt unter Kühlung mit Ethylacetat, dann mit 32%iger Natronlauge, arbeitet wie üblich auf und erhält p-Fluor-N-methyl-N-(1-phenyl-3-dimethylaminopropyl)-anilin. Dihydrochlorid, F. 204°.

### Beispiele 262 bis 300

Analog Beispiel 261 erhält man durch Reduktion der entsprechenden 3-Phenyl-3-N-methylanilinopropansäure-N,N-dimethylamide bzw. -N-methylamide die in den Beispielen 1 bis 4, 6 bis 20 sowie 41 bis 60 angegebenen Verbindungen.

Beispiel 301

Analog Beispiel 261 erhält man aus p-Trifluormethyl-N-methyl-N-[2-(1-methyl-6-oxo-2-piperidyl)-ethyl]-anilin [erhältlich aus p-Trifluormethyl-N-methyl-anilin und 2-(1-Methyl-6-oxo-2-piperidyl)-ethylchlorid analog Beispiel 1] mit $LiAlH_4$ das p-Trifluormethyl-N-methyl-N-[2-(1-methyl-2-piperidyl)-ethyl]-anilin, Rf 0,42.

Beispiele 302 bis 320

Analog Beispiel 301 erhält man aus den entsprechenden Piperidonderivaten mit $LiAlH_4$ die in den Beispielen 21 bis 37, 39 und 40 angegebenen Verbindungen.

Beispiel 321

Man löst 31,7 g p-Chlor-N-methyl-N-(1-phenyl-3-N-methylformamido-propyl)-anilin (erhältlich durch zweistündiges Kochen von p-Chlor-N-methyl-N-(1-phenyl-3-methyl-aminopropyl)-anilin mit Ethylformiat) in 600 ml THF und tropft diese Lösung unter Rühren zu einer Lösung von 3,8 g $LiAlH_4$ in 100 ml THF. Nach zweistündigem Rühren bei 20° wird gesättigte $Na_2SO_4$-Lösung zugegeben, der Niederschlag abfiltriert, das Filtrat eingeengt und wie üblich aufgearbeitet. Man erhält p-Chlor-N-methyl-N-(1-phenyl-3-dimethylaminopropyl)-anilin. Dihydrochlorid, F. 164°.

Beispiele 322 bis 360

Analog Beispiel 321 erhält man durch Reduktion der entsprechenden N-Methyl-N-(1-phenyl-3-formamidopropyl)-aniline die in den Beispielen 2 bis 20 sowie 41 bis 60 angegebenen Verbindungen.

Beispiel 361

Man löst 1 g 1-Methyl-2-[2-(p-methoxy-N-methylanilino)-ethyl]-pyridiniumchlorid [erhältlich durch Reaktion von p-Methoxy-N-methylanilin mit 1-Methyl-2-(2-chlorethyl)-pyridiniumchlorid] in 25 ml Methanol, hydriert an 0,1 g Platin bei 25° und 1 bar bis zum Stillstand, filtriert, dampft ein und erhält nach üblicher Aufarbeitung p-Methoxy-N-methyl-N-[2-(1-methyl-2-piperidyl)-ethyl]-anilin. Hydrochlorid, F. 138°.

Beispiele 362 bis 380

Analog Beispiel 361 erhält man aus den entsprechenden Pyridiniumchloriden durch Hydrierung die in den Beispielen 21 bis 29 und 31 bis 40 angegebenen Verbindungen.

Beispiel 381

Eine Lösung von 30,6 g 1-Dimethylamino-3-phenyl-3-N-methyl-N-(5-indanyl)-amino-propen (erhältlich durch Reaktion von 5-Methylaminoindan mit Zimtaldehyd zu 3-Phenyl-3-N-methyl-N-(5-indanyl)-amino-propanal und Umsetzung mit Dimethylamin) in 250 ml Dioxan wird an 2 g 2%igem Pd-C bei 20° und Normaldruck bis zur Aufnahme von 1 Mol Wasserstoff hydriert. Man filtriert, dampft ein und erhält 5-(N-Methyl-N-1-phenyl-3-dimethylamino-propylamino)-indan. Dihydrochlorid, F. 184°.

Beispiele 382 bis 420

Analog Beispiel 381 erhält man durch Hydrierung der entsprechenden 1-Dimethylamino- bzw. 1-Methylamino-3-phenyl-3-N-methyl-anilino-propene die in den Beispielen 1 bis 19 sowie 41 bis 60 angegebenen Verbindungen.

Beispiel 421

Man löst 31,7 g p-Chlor-N-methyl-N-[1-phenyl-3-(N-formyl-N-methyl-amino)-propyl]-anilin [erhältlich durch Umsetzung von N-Methyl-N-(1-chlor-3-phenylpropyl)-formamid mit p-Chlor-N-methyl-anilin analog Beispiel 1] in 320 ml einer 5%igen Lösung von KOH in 90%igem Ethanol, kocht 5 Stunden, dampft ein, arbeitet wie üblich auf und erhält p-Chlor-N-methyl-N-(1-phenyl-3-methylaminopropyl)-anilin.

Beispiele 422 bis 440

Analog Beispiel 421 erhält man durch alkalische Hydrolyse der entsprechenden N-Formylverbindungen die in den Beispielen 41 bis 46 sowie 48 bis 60 angegebenen Verbindungen.

Beispiel 441

Analog Beispiel 141 erhält man aus 1-Methylamino-3-phenyl-3-(p-fluor-N-methylanilino)-propan-hydrochlorid und Ameisensäure/Natriumformiat/Formaldehyd das p-Fluor-N-methyl-N-(1-phenyl-3-dimethylaminopropyl)-anilin. Dihydrochlorid, F. 204°.

Beispiele 442 bis 460

Analog Beispiel 441 erhält man aus den entsprechenden 1-Methylamino-3-phenyl-3-N-methylanilino-propanhydrochloriden mit Ameisensäure/Natriumformiat/Formaldehyd die in den Beispielen 1 bis 4 und 6 bis 20 beschriebenen Verbindungen.

Beispiel 461

Ein Gemisch aus 1 g p-Methoxy-N-methyl-N-(1-phenyl-3-dimethylaminopropyl)-anilin-hydrochlorid und 1 g Pyridinhydrochlorid wird 3 Stunden bei 160° gerührt. Nach üblicher Aufarbeitung erhält man p-Hydroxy-N-methyl-N-(1-phenyl-3-dimethylamino-propyl)-anilin.

Beispiel 462

Ein Gemisch aus 24,8 g p-Hydroxy-N-methyl-N-[2-(1-methyl-2-piperidyl)-ethyl]-anilin, 100 ml absolutem Ethanol und 200 ml 0,5 n ethanolischer KOH wird eine Stunde bei 20° gerührt und eingedampft. Man löst den Rückstand in 250 ml absolutem DMF und versetzt portionsweise unter Rühren mit 12,6 g Dimethylsulfat. Man kocht das Gemisch zwei Stunden, dampft ein, arbeitet wie üblich auf und erhält p-Methoxy-N-methyl-N-[2-(1-methyl-2-piperidyl)-ethyl]-anilin. Hydrochlorid, F. 138°.

Die nachstehenden Beispiele betreffen pharmazeutische Zubereitungen, die Amine der Formel I oder ihre Säureadditionssalze enthalten.

Beispiel A

Tabletten

Ein Gemisch von 1 kg p-Chlor-N-methyl-N-(1-phenyl-3-dimethylamino-propyl)-anilin-hydrochlorid, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 10 mg Wirkstoff enthält.

Beispiel B

Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C

Kapseln

2 kg p-Chlor-N-methyl-N-(1-phenyl-3-dimethylamino-propyl)-anilin-hydrochlorid werden in üblicher Weise in Hartgelatinekapseln gefüllt, so daß jede Kapsel 20 mg des Wirkstoffs enthält.

Beispiel D

Ampullen

Eine Lösung von 1 kg p-Chlor-N-methyl-N-(1-phenyl-3-dimethylamino-propyl)-anilin-hydrochlorid in 30 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

**Patentansprüche**

1. Anilinderivate der allgemeinen Formel I

$$Ar-N(CH_3)-R \qquad I$$

worin

Ar Phenyl oder eine durch F, Cl, Alkoxy mit bis zu 4 C-Atomen, Methylendioxy, OH, Trimethylen oder $CF_3$ substituierte Phenylgruppe,
R $-CH(C_6H_5)-CH_2CH_2-N(CH_3)-Z$ oder 2-(1-Methyl-2-piperidyl)-ethyl und
Z H oder Methyl

bedeuten,
sowie deren physiologisch unbedenklichen Säureadditionssalze.

2. p-Chlor-N-methyl-N-(1-phenyl-3-dimethylaminopropyl)-anilin.

3. Verfahren zur Herstellung von Anilinderivaten der allgemeinen Formel I

$$Ar-N(CH_3)-R \qquad I$$

worin

Ar Phenyl oder eine durch F, Cl, Alkoxy mit bis zu 4 C-Atomen, Methylendioxy, OH, Trimethylen oder $CF_3$ substituierte Phenylgruppe,
R $-CH(C_6H_5)-CH_2CH_2-N(CH_3)-Z$ oder 2-(1-Methyl-2-piperidyl)-ethyl und
Z H oder Methyl

bedeuten,
sowie von deren physiologisch unbedenklichen Säureadditionssalzen, dadurch gekennzeichnet, daß man ein Amin der allgemeinen Formel II

$$Ar-NHCH_3 \qquad II$$

worin

Ar die angegebene Bedeutung hat,

oder eines seiner Salze mit einem Amin der allgemeinen Formel III

$$X-R \qquad III$$

worin

X Cl, Br, J oder OH bedeutet und
R die angegebene Bedeutung hat,

oder mit einem seiner reaktionsfähigen Derivate umsetzt
oder daß man ein Amin der allgemeinen Formel IV

$$Ar-Y \qquad IV$$

worin

Y —NH—R, N-Methyl-N-(1-phenyl-3-aminopropyl)-amino oder N-Methyl-N-[2-(2-piperidyl)-ethyl]-amino bedeutet und
Ar und R die angegebenen Bedeutungen haben,

methyliert
oder daß man ein Amin der allgemeinen Formel V

$$Ar-N(CH_3)-CH(C_6H_5)-CH_2-CH_2-X \qquad V$$

worin

Ar und X die angegebenen Bedeutungen haben,

oder eines seiner reaktionsfähigen Derivate mit einem Amin der allgemeinen Formel VI

$$H-N(CH_3)-Z \qquad VI$$

worin

Z die angegebene Bedeutung hat,

umsetzt
oder daß man eine Verbindung, die der allgemeinen Formel I entspricht, jedoch an Stelle von Wasserstoffatomen eine oder mehrere reduzierbare Gruppen und/oder eine oder mehrere C—C- und/oder C—N-Mehrfachbindungen enthält, mit einem reduzierenden Mittel behandelt
oder daß man eine Verbindung der allgemeinen Formel VII

$$Ar-N(CH_3)-CH(C_6H_5)-CH_2-CH_2-N(CH_3)-W \qquad VII$$

worin

W eine Aminoschutzgruppe bedeutet und
Ar die angegebene Bedeutung hat,

mit einem solvolysierenden Mittel behandelt
und/oder daß man gegebenenfalls in einer Verbindung der Formel I eine NH-Gruppe zur $N(CH_3)$-Gruppe methyliert und/oder eine Hydroxygruppe durch Behandeln mit einem alkylierenden Mittel in die entsprechende Alkoxygruppe umwandelt und/oder eine Alkoxygruppe unter Bildung einer Hydroxygruppe spaltet und/oder eine erhaltene Base der Formel I durch Behandeln mit einer Säure in eines ihrer physiologisch unbedenklichen Säureadditionssalze umwandelt.

4. Verfahren zur Herstellung pharmazeutischer Zubereitungen, dadurch gekennzeichnet, daß man eine Verbindung der Formel I und/oder eines ihrer physiologisch unbedenklichen Säureadditionssalze zusammen mit mindestens einem festen, flüssigen oder halbflüssigen Träger- oder Hilfsstoff und gegebenenfalls in Kombination mit einem oder mehreren weiteren Wirkstoffen in eine geeignete Dosierungsform bringt.

5. Pharmazeutische Zubereitung, gekennzeichnet durch einen Gehalt an mindestens einer Verbindung der allgemeinen Formel I und/oder einem ihrer physiologisch unbedenklichen Säureadditionssalze.

## Claims

1. Aniline derivatives of the general formula I

$$Ar-N(CH_3)-R \qquad I$$

wherein Ar is phenyl or a phenyl group which is substituted by F, Cl, alkoxy with up to 4 C atoms, methylenedioxy, OH, trimethylene or $CF_3$, R is $-CH(C_6H_5)-CH_2CH_2-N(CH_3)-Z$ or

2-(1-methyl-2-piperidyl)-ethyl and Z is H or methyl, and physiologically acceptable acid addition salts thereof.

2. p-Chloro-N-methyl-N-(1-phenyl-3-dimethylamino-propyl)-aniline.

3. Process for the preparation of aniline derivatives of the general formula I

$$Ar-N(CH_3)-R \qquad I$$

wherein Ar is phenyl or a phenyl group which is substituted by F, Cl, alkoxy with up to 4 C atoms, methylenedioxy, OH, trimethylene or $CF_3$, R is $-CH(C_6H_5)-CH_2CH_2-N(CH_3)-Z$ or 2-(1-methyl-2-piperidyl)-ethyl and Z is H or methyl and of phisiologically acceptable acid addition salts thereof, characterised in that an amine of the general formula II

$$Ar-NHCH_3 \qquad II$$

wherein Ar has the meaning given, or one of its salts, is reacted with an amine of the general formula III

$$X-R \qquad III$$

wherein X is Cl, Br, I or OH and R has the meaning given, or with one of its reactive derivatives, or in that an amine of the general formula IV

$$Ar-Y \qquad IV$$

wherein Y is $-NH-R$, N-methyl-N-(1-phenyl-3-aminopropyl)-amino or N-methyl-N-[2-(2-piperidyl)-ethyl]-amino and Ar and R have the meanings given, is methylated, or in that an amine of the general formula V

$$Ar-N(CH_3)-CH(C_6H_5)-CH_2-CH_2-X \qquad V$$

wherein Ar and X have the meanings given, or one of its reactive derivatives, is reacted with an amine of the general formula VI

$$H-N(CH_3)-Z \qquad VI$$

wherein Z has the meaning given, or in that a compound which corresponds to the general formula I, but contains one or more reducible groups and/or one or more C–C- and/or C–N multiple bonds instead of hydrogen atoms, is treated with a reducing agent or in that a compound of the general formula VII

$$Ar-N(CH_3)-CH(C_6H_5)-CH_2-CH_2-N(CH_3)-W \qquad VII$$

wherein W is an amino-protecting group and Ar has the meaning given, is treated with a solvolyzing agent, and/or in that, in a compound of the formula I, if appropriate, an NH-group is methylated to yield an $N(CH_3)$-group and/or a hydroxyl group is vonverted, by treatment with an alkylating agent, into the corresponding alkoxy group, and/or an alkoxy group is split, a hydroxyl group being formed, and/or a resulting base of the formula I is converted into one of its physiologically acceptable acid addition salts by treatment with an acid.

4. Process for the preparation of pharmaceutical formulations, characterised in that a compound of the formula I and/or one of its physiologically acceptable acid addition salts is brought into a suitable dosage form together with at least one solid, liquid or semiliquid excipient or auxiliary, and if appropriate in combination with one or more other active compounds.

5. Pharmaceutical formulation, characterised in that it contains at least one compound of the general formula I and/or one of its physiologically acceptable acid addition salts.

**Revendications**

1. Dérivés d'aniline de formule générale I

$$Ar-N(CH_3)-R \qquad I$$

où

Ar représente un phényle ou un groupe phényle substitué par F, Cl, un alcoxy ayant jusqu'à 4 atomes de carbone, un méthylènedioxy, OH, un triméthylène ou $CF_3$,

R représente $-CH(C_6H_5)-CH_2CH_2-N(CH_3)-Z$ ou un 2-(1-méthyl-2-pipéridyl)-éthyle et
Z représente H ou un méthyle,

ainsi que leurs sels d'addition d'acides physiologiquement acceptables.

2. p-chloro-N-méthyl-N-(1-phényl-3-diméthylaminopropyl)-aniline.

3. Procédé de préparation de dérivés d'aniline de formule générale I

$$Ar-N(CH_3)-R \qquad I$$

où

Ar représente un phényle ou un groupe phényle substitué par F, Cl, un alcoxy ayant jusqu'à 4 atomes de carbone, un méthylènedioxy OH, un triméthylène ou $CF_3$,
R représente $-CH(C_6H_5)-CH_2CH_2-N(CH_3)-Z$ ou un 2-(1-méthyl-2-pipéridyl)-éthyle et
Z représente H ou un méthyle,

ainsi que de leurs sels d'addition d'acides physiologiquement acceptables, caractérisé en ce qu'on fait réagir une amine de formule générale II

$$Ar-NHCH_3 \qquad II$$

où

Ar a la signification donnée,

ou un de ses sels, avec une amine de formule générale III

$$X-R \qquad III$$

où

X représente Cl, Br, I ou OH et
R a la signification donnée ci-dessus,

ou avec un de ses dérivés réactifs,
ou en ce qu'on méthyle une amine de formule générale IV

$$Ar-Y \qquad IV$$

où

Y représente $-NH-R$, un N-méthyl-N-(1-phényl-3-aminopropyl)-amino ou un N-méthyl-N-[2-(2-pipéridyl)-éthyl]-amino et où
Ar et R ont les significations données,

ou en ce qu'on fait réagir une amine de formule générale V

$$Ar-N(CH_3)-CH(C_6H_5)-CH_2-CH_2-X \qquad V$$

où

Ar et X ont les significations données,

ou un de ses dérivés réactifs, avec une amine de formule générale VI

$$H-N(CH_3)-Z \qquad VI$$

où

Z a la signification donnée,

ou en ce qu'on traite un composé qui correspond à la formule générale I, mais qui contient à la place d'atomes d'hydrogène un ou plusieurs groupes réductibles et/ou une ou plusieurs liaisons multiples C—C et/ou C—N, avec un agent réducteur, ou en ce qu'on traite un composé de formule générale VII

$$Ar-N(CH_3)-CH(C_6H_5)-CH_2-CH_2-N(CH_3)-W \qquad VII$$

où

W représente un groupe protecteur d'amino et
Ar a la signification donnée,

avec un agent de solvolyse,
et/ou en ce que le cas échéant dans un composé de formule I on méthyle un groupe NH en groupe $N(CH_3)$ et/ou en ce qu'on transforme un groupe hydroxy par traitement avec un agent alcoylant en le groupe alcoxy correspondant et/ou en ce qu'on clive un groupe alcoxy avec formation d'un groupe hydroxy et/ou en ce qu'on transforme une base de formule I obtenue par traitement avec un acide en l'un de ces sels d'addition d'acides physiologiquement acceptables.

4. Procédé de préparation de préparations pharmaceutiques, caractérisé en ce qu'on amène sous une forme posologique appropriée un composé de formule I et/ou un de ses sels d'addition d'acides physiologiquement acceptables avec au moins un support ou additif solide, liquide ou semi-liquide et éventuellement en combinaison avec une ou plusieurs autres substances actives.

5. Préparation pharmaceutique caractérisée en ce qu'elle contient au moins un composé de formule générale I et/ou un de ses sels d'addition d'acides physiologiquement acceptables.